# EUROPEAN PATENT SPECIFICATION

(11) **EP 0 383 621 B1**
(45) Date of publication and mention of the grant of the patent: **08.12.1993**
(21) Application number: 90301712.7
(22) Date of filing: 16.02.1990
(51) Int. Cl.: C07C 43/225, C07C 211/52, C08F 138/00

(54) **Diphenyldiacetylene derivative**
Diphenyldiacetylenderivate
Dérivés de diphényldiacétylène

(30) Priority: 16.02.1989 JP 37020/89
(43) Date of publication of application: 22.08.1990
(73) Proprietor: TODA KOGYO CORP., Hiroshima-shi Hiroshima-ken (JP); The Agency of Industrial Science and Technology, Chiyoda-ku Tokyo-to (JP)
(72) Inventor: Matsuda, Hiro, Tsukuba-shi, Ibaraki-ken (JP); Okada, Shuji, Tsukuba-shi, Ibaraki-ken (JP); Nakanishi, Hachiro, Tsukuba-shi, Ibaraki-ken (JP); Kato, Masao, Tsukuba-shi, Ibaraki-ken (JP); Ohsugi, Minoru, Tsukuba-shi, Ibaraki-ken (JP); Takaragi, Shigeru, Hiroshima-shi, Hiroshima-ken (JP); Horiishi, Nanao, Hiroshima-shi, Hiroshima-ken (JP)
(74) Representative: Woods, Geoffrey Corlett

(56) References cited:
- EP-A- 0 243 807
- CHEMICAL ABSTRACTS, vol. 101, no. 4, 23rd July 1984, page 11, abstract no. 24111s, Columbus, Ohio, US; & JP-A-5946233
- CHEMTRONICS, vol. 3, December 1988, page 211-214, Bulterworth & CO. LTD; J. TSIBOUKLIS et al.: "Synthesis, polymerization and second order non-linear optical properties of novel, conjugated, unsymmetrical diacetylenes"
- CAREY, F.A. and SUNDBERG, R.J.: "Advanced Organic Chemistry" (1984), part A, p. 190
- OKADA, S. et al., Mol. Cryst. Liq. Cryst., 1990, vol. 183, pp. 81-90

## Description

The present invention relates to diphenyldiacetylene derivatives, their preparation and to polymerizing them.

The diphenyldiacetylene derivatives according to the present invention may be used as functional materials such as non-linear optical materials, photosensitive materials and semiconductive polymer crystals because they have solid-state polymerizability.

In recent years, research has been actively carried out on diacetylene compound serving as monomers used for forming, for example, non-linear optical materials, photosensitive materials and semiconductive polymer crystals.

Diacetylene monomers used for forming non-linear optical materials, photosensitive materials and semiconductive polymer crystals must have solid-state polymerizability. It has been also already known that, in order to improve the non-linear optical effect of non-linear optical materials, the monomers used must have an unsymmetrical structure and exhibit a large electronic effect owing to the conjugation bond between the main chain and the side chains.

It is generally known that some diacetylene compounds represented by the following formula (I) have solid-state polymerizability:

Rₐ - C ≡ C - C ≡ C - R_{b} (I)

There is now a strong demand for monomers having solid-state polymerizability, an unsymmetrical structure and substituents which are conjugated with the diacetylene triple bonds. However, as the above-described known diacetylene compounds having solid-state polymerizability have the same substituents at both ends of the diacetylene part, i.e., Rₐ = R_{b} in formula (I), to form a symmetric molecule, a charge-transfer effect cannot be expected.

In addition, most of such diacetylene compounds have a structure in which the conjugation bond between the substituents and the diacetylene part is cut off. Polymers, in which the conjugation bond between the main chain and the side chains is cut off, exhibit substantially no electronic effect of the substituents Rₐ and R_{b}, and any substituents in the polymers have the tendency to exhibit similar properties.

On the other hand, since diacetylene compounds represented by formula (I), having as the substituents Rₐ and R_{b} aromatic groups, have a conjugation bond between the triple bonds and the substituents, it is expected that the crystalline polymers formed have excellent electronic properties. However, most of such diacetylene compounds exhibit no solid-state polymerizability.

Some diphenyldiacetylene compounds which have two phenyl groups each having acetylamino groups at the ortho and meta positions or two phenyl groups each having trifluoromethyl groups at the 2- and 4- positions, 2- and 5-positions or 3- and 5-positions, have solid-state polymerizability. However, these compounds have a symmetric molecular structure and thus exhibit no charge-transfer effect.

EP-A-243,807 describes diacetylene monomers of formula (I) wherein Rₐ and R_{b} are phenyl groups, one being para-substituted by an amino group itself disubstituted with alkyl groups and the other being para-substituted by NO₂, CN or CF₃. The only such monomer specifically mentioned in the Examples, wherein Rₐ is dimethylamino and R_{b} is NO₂, cannot be solid state polymerized.

Tsibouklis, J.et al, Chemtronics, 1988(3), December, pp 211-214 describes various diacetylene monomers of formula (I) wherein Rₐ and R_{b} are phenyl groups with various substituents. Only one of these can be solid state polymerized. As described above, diacetylene monomers which have solid-state polymerizability, an unsymmetrical structure and aromatic groups as substitutents which are conjugated with the diacetylene triple bonds, and which thus exhibit a large electronic effect, have been strongly demanded.

As the results of the inventors' studies for obtaining diacetylene monomers which have solid-state polymerizability, an unsymmetrical structure and aromatic groups as substituents which are conjugated with the diacetylene triple bonds and which thus exhibit a large electronic effect, it has been found certain diphenyldiacetylene derivatives have solid-state polymerizability and an unsymmetrical structure and exhibit a large electronic effect.

Accordingly the present invention provides a diphenyldiacetylene derivative represented by the following formula (II): 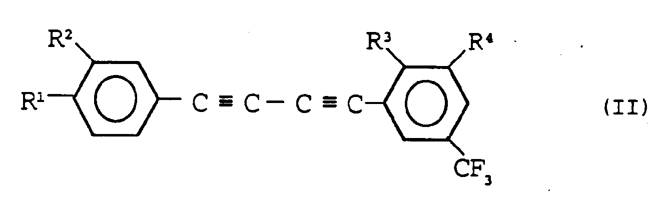
(wherein R¹ denotes a hydrogen atom or a methoxy group; R² denotes a hydrogen atom, a methoxy group or a methylamino group; one of R³ and R⁴ denotes a hydrogen atom and the other denotes a trifluoromethyl group; provided that R¹ is not a hydrogen atom when R² is a hydrogen atom).

The present invention also provides a process for producing a diphenyldiacetylene derivative represented by the formula (II), characterized in that:
an ethynylbenzene derivative represented by the following formula (VI): 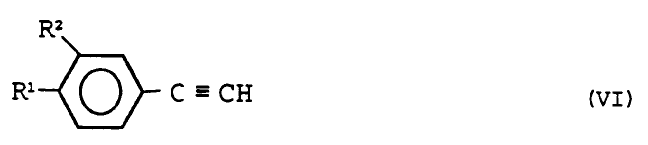
(wherein R¹ and R² are as defined above) and 1-bromo-3-hydroxy-3-methyl-1-butyne represented by the following formula (VII): 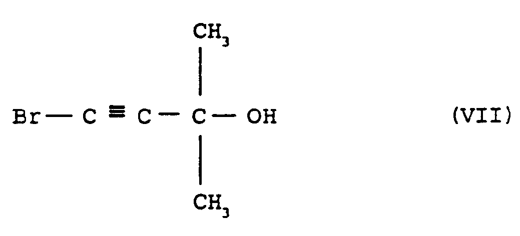
are subjected to unsymmetrical coupling reaction; the resultant diacetylene derivative represented by the following formula (VIII): 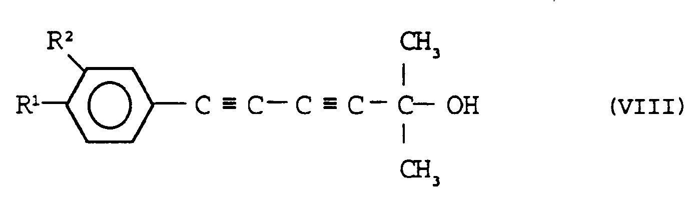
(wherein R¹ and R² each denote the same as that described above) is deacetonated in the presence of a basic catalyst; and
the resultant phenylbutadiyne derivative represented by the following formula (IX): 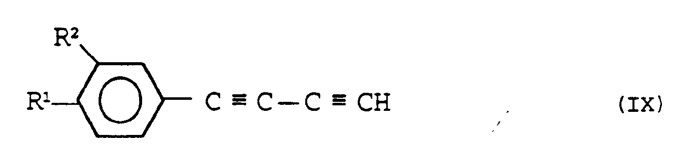
(wherein R¹ and R² each denote the same as that described above) and bis(trifluoromethyl) iodobenzene represented by the following formula (X): 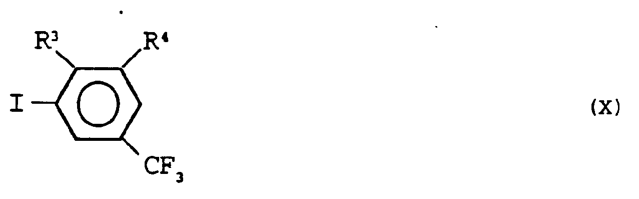
(wherein R³ and R⁴ each are as defined above) are subjected to coupling reaction in the presence of a palladium catalyst.

The diphenyldiacetylene derivatives represented by the formula (II) of the present invention are functional materials. Examples of the derivatives are:

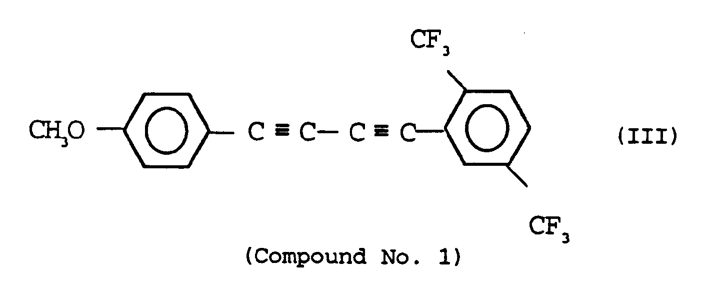

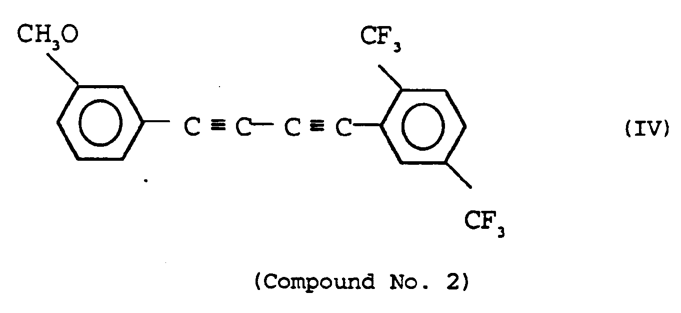

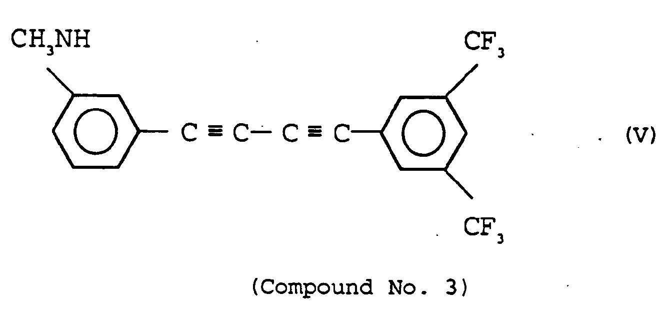

Each of the diphenyldiacetylene derivatives represented by the formula (II) of the present invention can produce non-linear optical materials, photosensitive materials and semiconductive polymer crystals owing to the solid-state polymerizability thereof, and exhibit a large non-linear optical effect owing to the large electronic effect caused by the unsymmetrical structure and the aromatic substituents which are conjugated with the diacetylene triple bonds.

Each of the diphenyldiacetylene derivatives represented by the formula (II) of the present invention has trifluoromethyl groups (-CF₃) serving as electron attractive groups and methoxy groups or N-methylamino groups serving as electron donative groups and is a compound which has a significantly polarized molecule. Therefore, a large non-linear optical effect can be expected, and the derivatives are significantly effective for use as non-linear materials.

A process for producing diphenyldiacetylene derivatives represented by the formula (II) of the present invention is set forth below:

Ethynylbenzene derivatives represented by the following formula (VI): 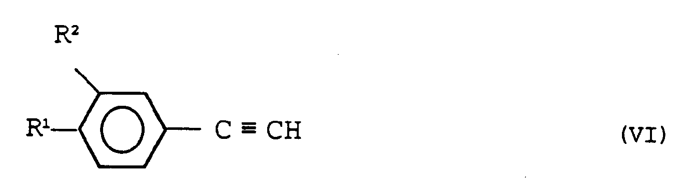
(wherein R¹ and R² are as defined above) and 1-bromo-3-hydroxy-3-methyl-1-butyne represented by the following formula (VII): 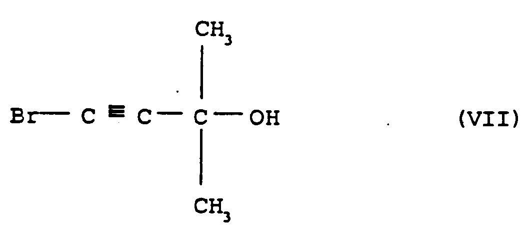
are subjected to unsymmetrical coupling reaction. The resultant diacetylene derivatives represented by the following formula (VIII): 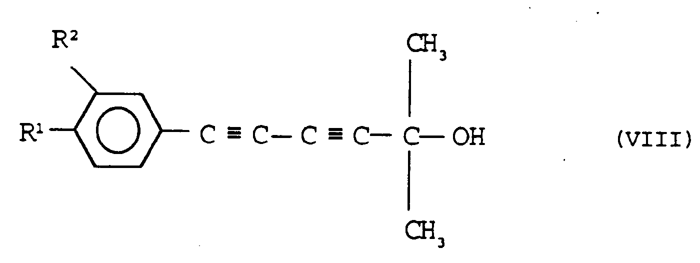
(wherein R¹ and R² are as defined above) are deacetonated in the presence of a basic catalyst. The resultant phenylbutadiyne derivatives represented by the following formula (IX): 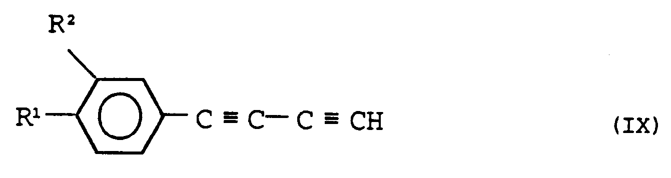
(wherein R¹ and R² are as defined above) and bis(trifluoromethyl) iodobenzene represented by the following formula (X): 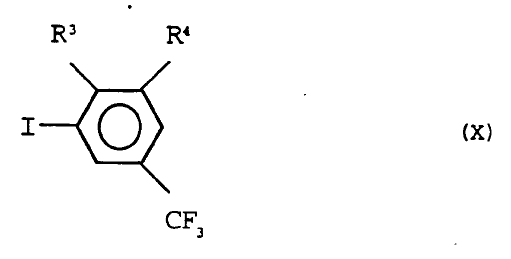
(wherein R³ and R⁴ are as defined above) are subjected to coupling reaction in the presence of a palladium catalyst to form the diphenyldiacetylene derivatives represented by the formula (II).

Although the unsymmetrical coupling reaction of acetylene derivatives can be carried out under various reaction conditions, the catalytic function of a copper salt is required in any case. Specifically, the unsymmetrical coupling reaction is carried out by adding an amine and copper (I) chloride to a solution of an ethynylbenzene derivative (ethynylanisole) and then slowly adding 1-bromo-3-hydroxy-3-methyl-1-butyne to the reaction mixture under stirring.

A polar solvent such as methanol, ethanol, tetrahydrofuran, dimethylformamide or dimethylacetamide is preferably used as a solvent. An aqueous ethylamine solution is generally used as the amine, and isopropylamine, for example, can be used when the reaction is carried out in a hydrophobic system. The copper (I) chloride is used in an amount of from 1 to 50 mol% based on the amount of ethynylaniline used.

This reaction is performed at 0 to 50°C in an atmosphere of an inert gas such as argon or nitrogen. In some cases, the reaction is performed for several hours to two days while an appropriate amount of hydroxylamine hydrochloride is added under stirring to prevent oxidation of copper (I) ions.

After the reaction has been completed, the solvent is evaporated from the reaction mixture, and the residue is neutralized and diluted by an aqueous solution of diluted hydrochloric acid and then subjected to extraction with a solvent such as ether or benzene . The solution obtained is dehydrated by adding a drying agent such as sodium sulfate or magnesium sulfate thereto, and the drying agent and the solvent are then removed. The residue is purified by silica-gel column chromatography to obtain a diacetylene derivative represented by the formula (VIII).

The thus-obtained diacetylene derivative represented by the formula (VIII) is then dissolved in benzene and refluxed while being heated for 30 min. to 1 hour in the presence of potassium hydroxide. After the reaction has been completed, the reaction mixture is filtered, and the solvent is then evaporated. The residue is then purified by silica-gel column chromatography to obtain a phenylbutadiyne derivative represented by the formula (IX).

The coupling reaction between the thus-formed phenylbutadiyne derivative and an aryl iodide represented by the formula (X) can be under various reaction conditions. In any case, the catalytic function of palladium (0) is required.

Specifically, the reaction is progressed by dissolving bis(trifluoromethyl) iodobenzene and the phenylbutadiyne derivative represented by the formula (IX) in the amine solvent and adding bis(triphenylphosphine) palladium (II) chloride to the resultant solution.

Triethylamine, diethylamine can, for example, be used as the solvent and, when the aryl iodide represented by the formula (X) or the phenylbutadiyne derivative represented by the formula (IX) is slightly soluble in the solvent, a solvent mixture of benzene or toluene and triethylamine or diethylamine can be used.

In the coupling reaction of the diphenyldiacetylene derivative, the reaction is carried out in an atmosphere of an inert gas such as argon or nitrogen, and a reaction initiator such as copper (I) iodide or copper (I) chloride may be added thereto.

After the reaction has been completed, the precipitate produced is filtered off from the reaction mixture, the filtrate is concentrated, neutralized and diluted with diluted hydrochloric acid, and then subjected to extraction with a solvent such as ether or benzene. The organic layer is dehydrated by a drying agent such as sodium sulfate or magnesium sulfate, and the solvent is then removed. The residue is then purified by silica-gel column chromatography to obtain a diphenyldiacetylene derivative represented by the formula (II).

Since each of the diphenyldiacetylene derivatives according to the present invention has an unsymmetrical structure and aromatic substituents which are conjugated with the diacetylene triple bonds, they have a non-linear optical effect. Since the derivatives have also solid-state polymerizability, they are suitable as diacetylene monomers for forming non-linear optical materials, photosensitive materials and semiconductive polymer materials.

The present invention is now further described in the following Examples.

### Example 1

100 mg of copper (I) chloride was dissolved in a mixture of 20 ml of isopropylamine and 20 ml of methanol in an atmosphere of argon, and 1320 mg of 4-ethynylanisole were added to the resultant solution, followed by stirring at room temperature for 10 minutes. 2700 mg of 1-bromo-3-hydroxy-3-methyl-1-butyne were then dropwisely added to the solution over a time of about 3 hours, followed by stirring at room temperature for 2 hours. If the solution is colored green owing to the production of copper (II) ions, an appropriate amount of hydroxylamine hydrochloride is added so as to reduce the copper (II) ions. The solvent was then evaporated from the reaction mixture, the residue was neutralized and diluted by 1N hydrochloric acid, and then subjected to extraction with 50 ml of dichloromethane three times. The organic layer was dried over magnesium sulfate, and the solvent was then evaporated. The residue was then purified by silica-gel column chromatography using benzene as an eluent to obtain 1500 mg of 5-hydroxy-5-methyl-1-(4-methoxyphenyl)hexa-1,3-diyne.

1500 mg of the diacetylene compound obtained were then dissolved in 300 ml of benzene, and 600 mg of potassium hydroxide, which were finely ground, was then added to the reaction mixture, followed by reflux under heating for 30 minutes. After filtration, the solvent was evaporated, and the residue was then purified by silica-gel chromatography using hexane as an eluent to obtain 1150 mg of 1-(4-methoxyphenyl)-1,3-butadiyne. This compound was a slightly yellowish white crystal.

1100 mg of the 1-(4-methoxyphenyl)-1,3-butadiyne obtained and 2700 mg of 2,5-bis(trifluoromethyl) iodobenzene were then dissolved in 50 ml of triethylamine, and 50 mg of bis(triphenylphosphine)palladium (II) chloride and 14 mg of copper (I) chloride were then rapidly added to the reaction mixture. After the solution had been stirred at room temperature for 13 hours, 50 ml of benzene were added to the reaction mixture, and the precipitate produced was then filtered off. After the solvent had been evaporated, the residue was neutralized by 1N hydrochloric acid and subjected to extraction with 50 ml of benzene three times. The organic layer was dried over magnesium sulfate, and the solvent was then evaporated. The residue was purified by silica-gel column chromatography using hexane as an eluent to obtain 1600 mg of 1-(4-methoxyphenyl)-4-[2,5-bis(trifluoromethyl)phenyl]-1,3-butadiyne. This compound was a needle-like white crystal having a melting point of from 86 to 88°C.
- Anal. Calc'd For (C₁₉H₁₀F₆O):: C, 61.97%, H, 2.74%
- Found:: C, 61.68%, H, 2.98%

### Example 2

50 mg of copper (I) chloride were dissolved in a mixture of 10 ml of isopropylamine and 15 ml of methanol in an atmosphere of argon, and 660 mg of 3-ethynylanisole were then added to the reaction mixture, followed by stirring at room temperature for 10 minutes. 1300 mg of 1-bromo-3-hydroxy-3-methyl-1-butyne were then dropwisely added to the solution over about 2 hours, followed by stirring at room temperature for 2 hours. The solvent was then evaporated from the reaction mixture, the residue was then neutralized and diluted by 1N hydrochloric acid, and subjected to extraction with dichloromethane three times. The organic layer was dried over magnesium sulfate, and the solvent was then evaporated. The residue was then purified by silica-gel column chromatography using benzene as an eluent to obtain 800 mg of 5-hydroxy-5-methyl-1-(3-methoxyphenyl)hexane-1,3-diyne.

800 mg of the thus-obtained diacetylene compound were dissolved in 200 ml of benzene, and 300 mg of potassium hydroxide were added to the reaction mixture, followed by reflux under heating for 15 minutes. After filtration, the solvent was evaporated, and the residue was purified by silica-gel chromatography using as hexane as an eluent to obtain 430 mg of 1-(3-methoxyphenyl)-1,3-butadiyne.

430 mg of the thus-obtained 1-(3-methoxyphenyl)-1,3-butadiyne and 1200 mg of 2,5-bis(trifluoromethyl) iodobenzene were dissolved in 20 ml of triethylamine in an atmosphere of argon, and 21 mg of bis(triphenylphosphine) palladium (II) chloride and 6 mg of copper (I) chloride were rapidly added to the reaction mixture. After the solution had been stirred at room temperature for 13 hours, 30 ml of benzene were added thereto, and the precipitate produced was filtered off. After the solvent had been evaporated, the residue was neutralized by 1N hydrochloric acid and then subjected to extraction with 50 ml benzene three times. The organic layer was dried over magnesium sulfate, and the solvent was then evaporated. The residue was then purified by silica-gel column chromatography using hexane as an eluent to obtain 300 mg of 1-(3-methoxyphenyl)-4-[2,5-bis(trifluoromethyl)phenyl]-1,3-butadiyne. This compound was a slightly yellowish white crystal having a melting point of 37°C.
- Anal. Calc'd For (C₁₉H₁₀F₆O):: C, 61.97%, H, 2.74%
- Found:: C, 61.72%, H, 2.85%

### Example 3

100 mg of copper (I) chloride were dissolved in a mixture of 20 ml of isopropylamine and 10 ml of methanol in an atmosphere of argon, and 1350 mg of 3-ethynyl-N-methylaniline were then added to the reaction mixture, followed by stirring at room temperature for 10 minutes. 3300 mg of 1-bromo-3-hydroxy-3-methyl-1-butyne were then dropwisely added to the solution over a time of about 3 hours, followed by stirring at room temperature for 2 hours. After the solvent had been evaporated from the reaction mixture, the residue was neutralized and diluted by 1N hydrochloric acid and subjected to extraction with 50 ml of ether three times. The organic layer was dried over magnesium sulfate, and the solvent was then evaporated. The residue was then purified by silica-gel chromatography using benzene as an eluent to obtain 1600 mg of 5-hydroxy-5-methyl-1-[3-(N-methylamino)phenyl]hexa-1,3-diyne.

1600 mg of the thus-obtained diacetylene compound were then dissolved in 300 ml of benzene, and 450 mg of potassium hydroxide were then added to the reaction mixture, followed by reflux under heating for 1 hour. After filtration, the solvent was evaporated, and the residue was then purified by silica-gel column chromatography using benzene as an eluent to obtain 500 mg of 1-[3-(N-methylamino)phenyl]-1,3-butadiyne.

320 mg of the thus-obtained 1- [3-(N-methylamino)phenyl]-1,3-butadiyne and 700 mg of 3,5-bis(trifluoromethyl) iodobenzene were dissolved in 30 ml of trimethylamine in an atmosphere of argon, and 14 mg of bis(triphenylphosphine)palladium (II) chloride and 4 mg of copper (I) chloride were then rapidly added to the reaction mixture. After the solution had been stirred at room temperature for 13 hours, 50 ml of benzene were added thereto, and the solvent was then evaporated. The residue was neutralized by 1N hydrochloric acid and subjected to extraction with 50 ml of benzene three times. The organic layer was then dried over magnesium sulfate, and the solvent was evaporated. The residue was then purified by silica-gel chromatography using a mixture of hexane and benzene as an eluent to obtain 580 mg of 1-[3-(N-methylamino)phenyl]-4-[3,5-bis(trifluoromethyl)phenyl]-1,3-butadiyne. This compound was a yellowish green crystal having a melting point of from 75 to 76°C.
- Anal. Calc'd For (C₁₉H₁₁F₆N):: C, 62.13%; H, 3.02%;
N, 3.81%
- Found:: C, 61.92%; H, 3.14%;
N, 4.03%

### Reference Example

A powder sample of each of the compounds of the present invention was loaded into a glass tube under vacuum and irradiated with cobalt-60 γ-rays at a dose of 300 MRAD so as to be solid-state polymerized. After the glass tube had been opened, the polymerization yield was determined from the weight of the polymer which was insoluble in benzene. The yields for all the samples were 100%.

## Claims

1. A diphenyldiacetylene derivative represented by the following formula (II): 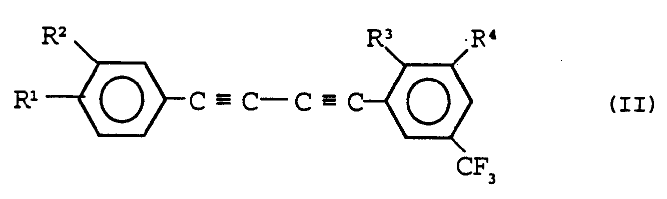 (wherein R¹ denotes a hydrogen atom or a methoxy group; R² denotes a hydrogen atom, a methoxy group or a methylamino group; one of R³ and R⁴ denotes a hydrogen atom and the other denotes a trifluoromethyl group; provided that R¹ is not a hydrogen atom when R² is hydrogen atom).

2. A diphenyldiacetylene derivative according to Claim 1, wherein R¹ denotes a methoxy group; R² and R⁴ each denote a hydrogen atom; and R³ denotes a trifluoromethyl group.

3. A diphenyldiacetylene derivative according to Claim 1, wherein R¹ and R⁴ each denote a hydrogen atom; R² denotes a methoxy group; and R³ denotes a trifluoromethyl group.

4. A diphenyldiacetylene derivative according to Claim 1, wherein R¹ and R³ each denote a hydrogen atom; R² denotes a methylamino group; and R⁴ denotes a trifluoromethyl group.

5. A process for producing a diphenyldiacetylene derivative represented by the formula (II), characterized in that
an ethynylbenzene derivative represented by the following formula (VI): 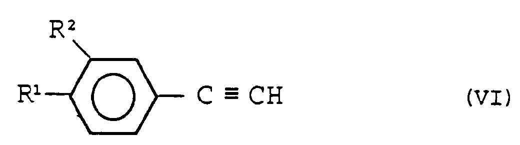 (wherein R¹ and R² are as defined in claim 1) and 1-bromo-3-hydroxy-3-methyl-1-butyne represented by the following formula (VII): 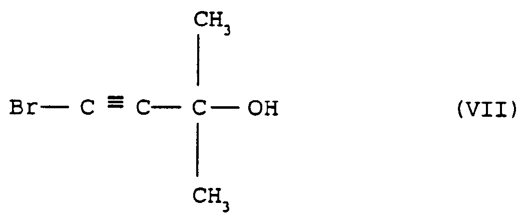 are subjected to unsymmetrical coupling reaction; the resultant diacetylene derivative represented by the following formula (VIII): 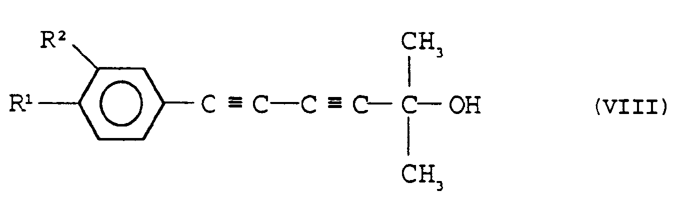 (wherein R¹ and R² each denote the same as that described above) is deacetonated in the presence of a basic catalyst; and
the resultant phenylbutadiyne
derivative represented by the following formula (IX): 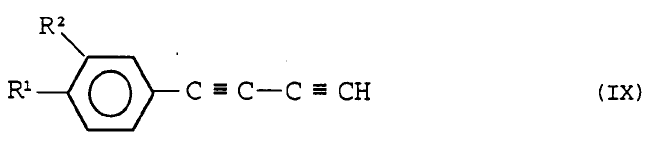 (wherein R¹ and R² each denote the same as that described above) and bis(trifluoromethyl) iodobenzene represented by the following formula (X): 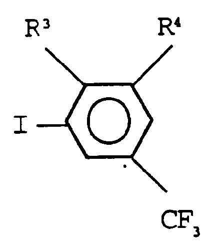 (wherein R³ and R⁴ are as defined in claim 1) are subjected to coupling reaction in the presence of a palladium catalyst.

6. A polymerization process, which process comprises polymerizing in the solid state a diphenyldiacetylene derivative of formula (II) as defined in claim 1.

## Patentansprüche

1. Diphenyldiacetylenderivat, dargestellt durch die folgende Formel (II): 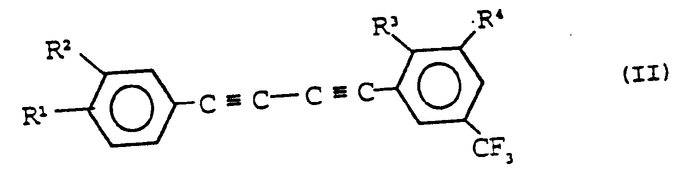 worin R₁ ein Wasserstoffatom oder eine Methoxygruppe ist; worin R² ein Wasserstoffatom, eine Methoxygruppe oder eine Methylaminogruppe ist; worin eines von R³ und R⁴ ein Wasserstoffatom und das andere eine Trifluoromethylgruppe ist; mit dem Vorbehalt, daß R¹ kein Wasserstoffatom ist, wenn R² ein Wasserstoffatom ist.

2. Diphenyldiacetylenderivat nach Anspruch 1,
dadurch **gekennzeichnet,** daß
R¹ eine Methoxygruppe; R² und R⁴ jeweils ein Wasserstoffatom; und R³ eine Trifluoromethylgruppe sind.

3. Diphenyldiacetylenderivat nach Anspruch 1,
dadurch **gekennzeichnet,** daß
R¹ und R⁴ jeweils ein Wasserstoffatom sind, R² eine Methoxygruppe und R³ eine Trifluoromethylgruppe ist.

4. Diphenyldiacetylenderivat nach Anspruch 1,
dadurch **gekennzeichnet,** daß
R¹ und R³ jeweils ein Wasserstoffatom sind; R² eine Methylaminogruppe ist und R⁴ eine Trifluoromethylgruppe ist.

5. Verfahren zur Herstellung eines Diphenyldiacetylenderivates, dargestellt durch die Formel (II), dadurch **gekennzeichnet,** daß
ein Ethynylbenzolderivat, dargestellt durch die folgende Formel (VI): 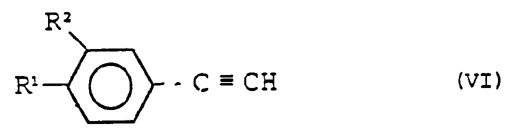 (Worin R¹ und R² wie in Anspruch 1 definiert sind), und 1-Bromo-3-hydroxy-3-methyl-1-butyn, dargestellt durch die folgende Formel (VII): 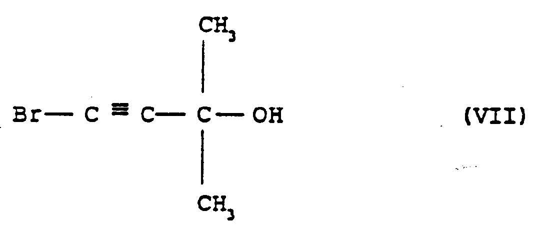 einer unsymmetrischen Kupplungsreaktion unterworfen werden; daß das resultierende Diacetylenderivat, dargestellt durch die folgende Formel (VIII): 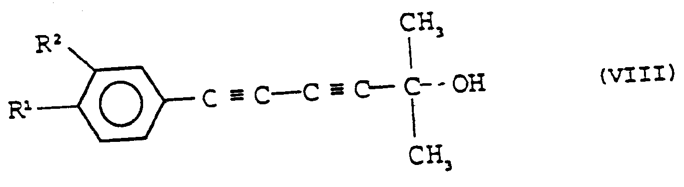 (worin R¹ und R² jeweils die gleiche Bedeutung aufweisen wie oben beschrieben) in der Gegenwart eines basischen Katalysators deacetoniert wird; und daß das resultierende Phenylbutadiynderivat, dargestellt durch die folgende Formel (IV): 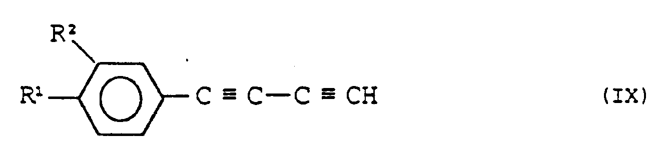 (worin R¹ und R² jeweils die gleichen Bedeutungen wie oben beschrieben aufweisen) und Bis(trifluoromethyl)iodobenzol, dargestellt durch die folgende Formel (X): 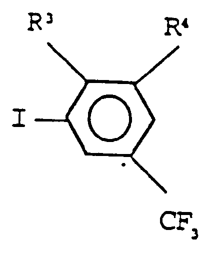 (worin R³ und R⁴ wie in Anspruch 1 definiert sind) einer Kupplungsreaktion in der Gegenwart eines Palladiumkatalysators unterworfen werden.

6. Polymerisationsverfahren, umfassend das Polymerisieren eines Diphenyldiacetylenderivates der Formel (II) nach Anspruch 1 in dem festen Zustand.

## Revendications

1. Dérivé de diphényldiacétylène représenté par la formule (II) : 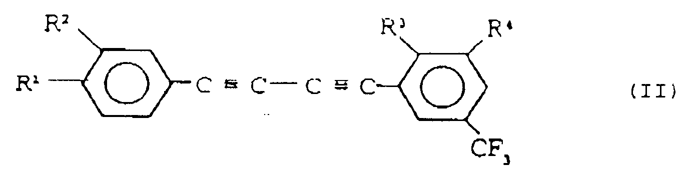 dans laquelle R¹ désigne un atome d'hydrogène ou un groupe méthoxy, R² désigne un atome d'hydrogène, un groupe méthoxy ou un groupe méthylamino, l'un des groupes R³ et R⁴ désigne un atome d'hydrogène et l'autre désigne un groupe trifluorométhyle, à condition que R¹ ne soit pas un atome d'hydrogène lorsque R² est un atome d'hydrogène.

2. Dérivé de diphényldiacétylène selon la revendication 1, dans lequel R¹ désigne un groupe méthoxy, R² et R⁴ désignent chacun un atome d'hydrogène et R³ désigne un groupe trifluorométhyle.

3. Dérivé de diphényldiacétylène selon la revendication 1, dans lequel R¹ et R⁴ désignent chacun un atome d'hydrogène, R² désigne un groupe méthoxy et R³ désigne un groupe trifluorométhyle.

4. Dérivé de diphényldiacétylène selon la revendication 1, dans lequel R¹ et R³ désignent chacun un atome d'hydrogène, R² désigne un groupe méthylamino et R⁴ désigne un groupe trifluorométhyle.

5. Procédé de préparation d'un dérivé de diphényldiacétylène représenté par la formule (II), caractérisé en ce qu'un dérivé d'éthynylbenzène représenté par la formule suivante (VI) : 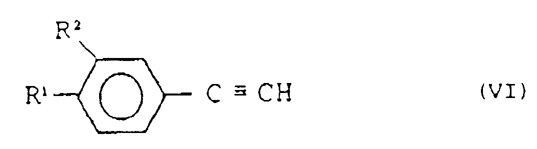 dans laquelle R¹ et R² sont tels que définis dans la revendication 1, et du 1-bromo-3-hydroxy-3-méthyl-1-butyne représenté par la formule suivante (VII) : 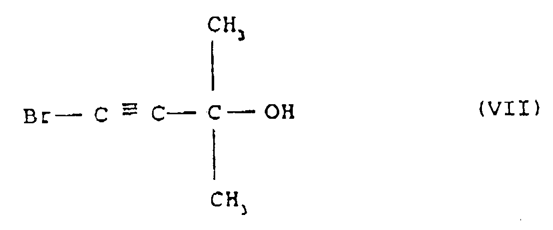 sont soumis à une réaction de couplage asymétrique; le dérivé de diacétylène obtenu représenté par la formule suivante (VIII) : 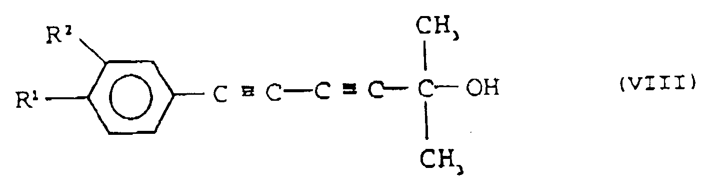 dans laquelle R¹ et R² désignent chacun les mêmes groupes que ceux décrit ci-dessus, est désacétoné en présence d'un catalyseur basique, et le dérivé de phénylbutadiyne obtenu représenté par la formule suivante (IX) : 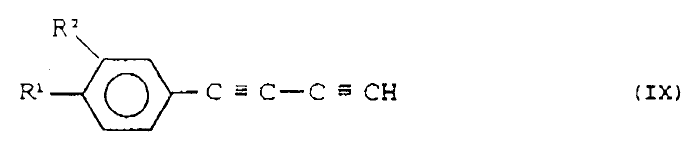 dans laquelle R¹ et R³ désignent chacun les mêmes groupes que ceux décrit ci-dessus, et du bis(trifluorométhyl)iodobenzène représenté par la formule suivante (X) : 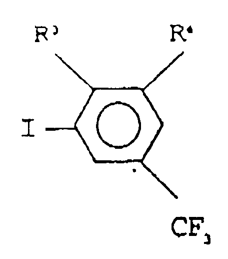 dans laquelle R³ et R⁴ sont tels que définis ci-dessus, sont soumis à une réaction de couplage en présence d'un catalyseur de palladium.

6. Procédé de polymérisation qui consiste à polymériser à l'état solide un dérivé de diphényldiacétylène de formule (II) selon la revendication 1.
